# EUROPEAN PATENT APPLICATION

(11) **EP 4 571 317 A1**
(43) Date of publication of application: **18.06.2025**
(21) Application number: 23848580.9
(22) Date of filing: 23.05.2023
(51) Int. Cl.: G01N 35/00

(54) **PARTS MANAGEMENT SYSTEM, AUTOMATIC ANALYZER, AND PARTS MANAGEMENT METHOD**

(30) Priority: 10.08.2022 JP 2022128086
(71) Applicant: HITACHI HIGH-TECH CORPORATION, Tokyo 105-6409 (JP)
(72) Inventor: MAEDA Shota, Tokyo 105-6409 (JP); YAMANO Teruhiro, Tokyo 105-6409 (JP)
(74) Representative: MERH-IP Matias Erny Reichl Hoffmann Patentanwälte PartG mbB
(86) International application number: PCT/JP2023/019187
(87) International publication number: WO 2024/034221

(57) **Abstract**

A part management system 1 includes: a display unit 14 that displays information about the part management system 1; an automatic analyzer storage unit 23 that stores, for each of the plurality of analysis devices 25, an ID of the corresponding analysis device 25 and state information indicating a state of the analysis device 25, the ID and the state information being stored in association with each other; an input unit 13 that receives a replacement request for the analysis device 25; and a part management apparatus control unit 11 that, when the input unit 13 receives a replacement request for the analysis device 25, causes the display unit 14 to display the ID of the analysis device 25 whose state information satisfies a predetermined requirement and first state information about a position where the analysis device 25 is used. Accordingly, a part management system, an automatic analyzer, and a part management method capable of supporting specification of a part to be removed even when parts of the same type are used in plurality of uses or positions are provided.

## Description

### Technical Field

The present invention relates to a part management system, an automatic analyzer, and a part management method.

### Background Art

Patent Literature 1 discloses a chromatography system that can analyze a sample under a plurality of analysis conditions. The chromatography system includes an apparatus body having a flow channel for passing a liquid sample, an analysis column disposed in the middle of the flow channel and configured to separate the sample into a plurality of components, a replaceable part attached to the apparatus body, and a storage unit storing first position information about an attachment position of the part to the apparatus body.

### Citation List

### Patent Literature

PTL 1: WO2020/183760

### Summary of Invention

### Technical Problem

In the above-described technique in the related art, an attachment position of a part and a name and a lot number of the part are stored in association with each other in a storage unit, and even when the same type of part is used in a plurality of uses or positions, attachment position information can be acquired by inputting a name or lot number of a part to be removed at the time of part replacement.

However, in a case where a part used in a plurality of uses or positions is to be replaced due to a failure or the like only by retrieval according to a name or lot number of the part, it is difficult to specify an actual replacement target part from removal candidate parts satisfying a retrieval requirement, and there is room for improvement.

In order to manage a replacement record of parts of an apparatus, even when the same type of part is used in a plurality of uses or positions in the apparatus, at the time of part replacement, it is necessary to specify one of the uses or positions of the part that needs to be removed for replacement, and to perform replacement and recording accurately.

Therefore, an object of the invention is to provide a part management system, an automatic analyzer, and a part management method capable of supporting specification of a part to be removed even when the same type of part is used in a plurality of uses or positions.

### Solution to Problem

The invention includes a plurality of means to solve the above problems, and an example thereof is a part management system for managing a plurality of parts used in an apparatus, the part management system including: a display unit that displays information about the part management system; a storage unit that stores, for each of the plurality of parts, an ID of the corresponding part and state information indicating a state of the part, the ID and the state information being stored in association with each other; a reception unit that receives a replacement request for the part; and a control unit that, when the reception unit receives a replacement request for the part, causes the display unit to display an ID of the part whose state information satisfies a predetermined requirement, and first state information about a position where the part is used.

### Advantageous Effects of Invention

According to the invention, even when the same type of part is used in a plurality of uses or positions, it is possible to support specification of a part to be removed. Problems, configurations, and effects other than those described above will become apparent in the following description of embodiments.

### Brief Description of Drawings

[FIG. 1] FIG. 1 is a diagram illustrating overall configuration of a part management system according to an embodiment.
[FIG. 2] FIG. 2 is a table illustrating an example of a data structure of a constituent part database in the part management system according to the embodiment.
[FIG. 3] FIG. 3 is a diagram illustrating an example of interfaces between part management systems in the part management system according to the embodiment.
[FIG. 4] FIG. 4 is a flowchart illustrating a processing procedure of a part management apparatus at a time of part replacement in the part management system according to the embodiment.
[FIG. 5] FIG. 5 is a diagram illustrating an example of record information in the part management system according to the embodiment.
[FIG. 6] FIG. 6 is a diagram illustrating an example of display of a display unit in the part management system according to the embodiment.
[FIG. 7] FIG. 7 is a diagram illustrating an example of the record information in a case where there are a plurality of removal part candidates in the part management system according to the embodiment.
[FIG. 8] FIG. 8 is a diagram illustrating an example of display of the display unit in a case where there are a plurality of removal part candidates in the part management system according to the embodiment.
[FIG. 9] FIG. 9 is a diagram illustrating an example of a screen for selecting state information to be displayed in the part management system according to the embodiment.

### Description of Embodiments

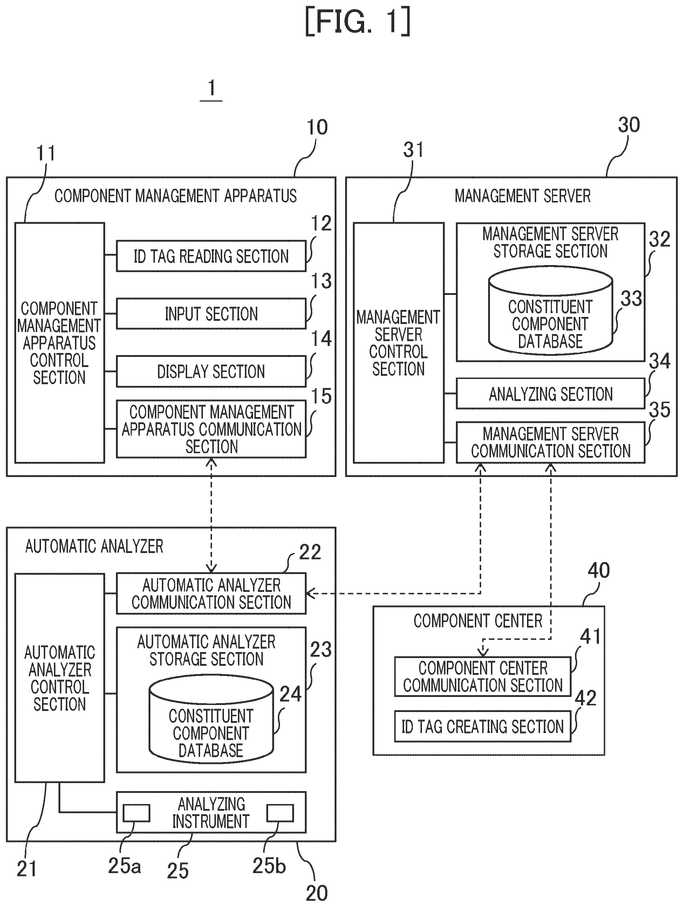

An embodiment of a part management system, an automatic analyzer, and a part management method according to the invention will be described with reference to FIGS. 1 to 9. In the following embodiment, it is needless to mention that components (also including element steps and the like) thereof are not necessarily essential unless otherwise specified or unless clearly considered to be essential in principle. In the drawings used in the description, the same or corresponding components are denoted by the same or similar reference signs, and repeated description of these components may be omitted.

First, an overall configuration of a part management system will be described with reference to FIG. 1. FIG. 1 illustrates an overall configuration of the part management system according to the embodiment.

In the embodiment, although a case will be described in which an apparatus is an automatic analyzer 20 that measures physical properties of a biological sample and a part is two or more analysis devices 25 used for the measurement of the physical properties, a target part of the part management system and the part management method according to the invention is not limited to an automatic analyzer.

A part management system 1 according to the invention illustrated in FIG. 1 is a system that manages a plurality of analysis devices 25 used in the automatic analyzer 20, and includes a part management apparatus 10, the automatic analyzer 20, a management server 30, and a part center 40. The part management apparatus 10 and the automatic analyzer 20, the automatic analyzer 20 and the management server 30, and the management server 30 and the part center 40 can communicate with each other with priority or wirelessly.

Although only one automatic analyzer 20 is shown, a plurality of automatic analyzers 20 may be provided.

The part management apparatus 10 includes a part management apparatus control unit 11, an ID tag read unit 12, an input unit 13, a display unit 14, and a part management apparatus communication unit 15, and is implemented by, for example, a portable personal computer owned by a service engineer of a manufacturer of the automatic analyzer 20 or the like in the embodiment.

The part management apparatus control unit 11 includes a CPU, a RAM, and a ROM, and can load a program stored in the ROM into the RAM and execute the program. For example, part record management described later that is performed in a constituent part database 24 of the automatic analyzer 20 at the time of part replacement is executed according to a program of the part management apparatus control unit 11. In addition, the part management apparatus control unit 11 is connected to other units, and can exchange data with the other units and relay data between the units. For example, information about an ID tag read by the ID tag read unit 12 is displayed on the display unit 14.

In the embodiment, when the input unit 13 receives a replacement request for the analysis device 25, the part management apparatus control unit 11 causes the display unit 14 to display an ID of the analysis device 25 whose state information satisfies a predetermined requirement and first state information about a position where the analysis device 25 is used. Further, the predetermined requirement may be a state ("state 3") in which preparation of a replacement product of the analysis device 25 is completed and the replacement is ready to be implemented. Details thereof will be described later.

The ID tag read unit 12 is a reading device for reading an ID tag attached to the analysis device 25 or a packaging material of the analysis device 25, and can output information read from the ID tag to the part management apparatus control unit 11.

The input unit 13 includes, in addition to an input device such as a keyboard and a mouse, a scanner that performs ID reading of the analysis device 25 to be replaced, and can transmit an input of a user to the part management apparatus control unit 11. In the embodiment, the input unit 13 receives a replacement request for the analysis device 25.

The display unit 14 is a display such as a liquid crystal display, and can display information about the part management system 1 that is transmitted from the part management apparatus control unit 11. The display unit 14 may be a touch panel type to serve as a part of the input unit 13.

The part management apparatus communication unit 15 can communicate with an automatic analyzer communication unit 22. For example, the part management apparatus communication unit 15 communicates with the automatic analyzer 20 to acquire information in the constituent part database 24 of the automatic analyzer 20.

The automatic analyzer 20 is an apparatus for causing a sample and a reagent to react with each other and measuring physical properties of a reacted reaction liquid, and includes an automatic analyzer control unit 21, an automatic analyzer storage unit 23 including the constituent part database 24, the automatic analyzer communication unit 22, and two or more analysis devices 25 used for measuring physical properties of a biological sample. A detailed configuration of the automatic analyzer 20 is not particularly limited, and may be various configurations such as a configuration in a single module, a configuration in a plurality of modules, a configuration in which a plurality of modules are coupled by a transport line or the like, and is not particularly limited.

The automatic analyzer control unit 21 includes a CPU, a RAM, and a ROM, and can load a program stored in the ROM into the RAM and execute the program. The automatic analyzer control unit 21 is connected to other units, and can exchange data with the other units and relay data between the units.

The automatic analyzer storage unit 23 is implemented by a storage medium such as a flash memory. The automatic analyzer storage unit 23 includes the constituent part database 24 that records information about one part (the analysis device 25) as one record.

In the embodiment, for each of a plurality of analysis devices 25, the automatic analyzer storage unit 23 stores an ID of the corresponding analysis device 25 and state information indicating a state of the analysis device 25, the ID and the state information being stored in association with each other.

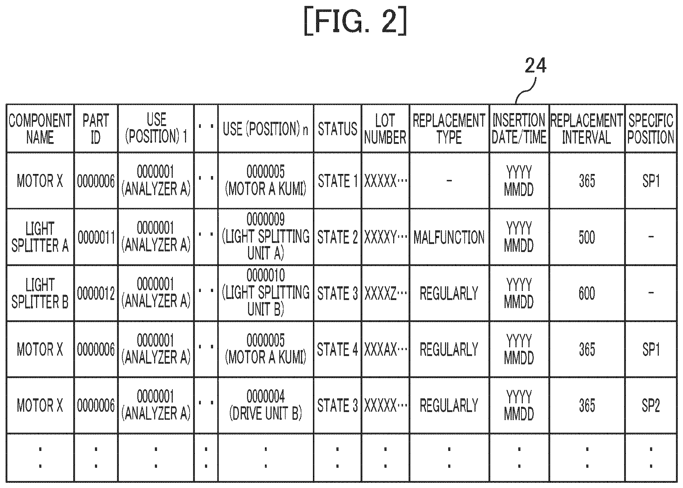

FIG. 2 illustrates an example of a data structure recorded in a part record in the constituent part database 24. In the example in FIG. 2, as part information, a "part name" indicating name information of a part, a "part ID" indicating ID information for specifying a type of the part, a "use or position" indicating use position information of the part in an apparatus, a "status" indicating state information of the part, a "lot number" indicating lot information of the part, a "replacement type" indicating replacement type information of the part, an "insertion date and time" indicating time information about a time when the part is assembled into the apparatus, an "replacement interval" indicating number-of-day information about how many days the part can be used are recorded.

As the use position information of the part, the "part name" and the "part ID" of all the parts corresponding to higher hierarchies of the part are basically recorded in a descending order from a part having the highest hierarchy.

However, when a plurality of parts of the same hierarchy and type are used, there is a possibility that use positions of the parts cannot be distinguished from each other by only part information of a higher hierarchy. Therefore, a "detailed position" indicating a detailed position of the part (record only for a plurality of parts having the same "use or position") is used, and for parts whose "use or position" coincide with each other, the "detailed position" and the "use or position" are combined to make it possible to distinguish a use position of the part. In addition, when it is desired to make a distinction only by the "use or position", the use position of the part can be distinguished by recording, as information of one hierarchy, detailed position information of the use position in place of the "part name" and ID information indicating the use position in place of the "part ID".

The state information of a part includes "state 1 (second state information)" of a state in which a part is normally assembled into an apparatus and the part is normally operating, "state 2 (third state information)" of a state in which a part assembled in the apparatus needs to be replaced and waits for a part arrangement, "state 3 (first state information)" of a state in which a part arrangement is completed and part replacement is possible, and "state 4 (fourth state information)" of a state in which a part is removed by part replacement and is not currently assembled into the apparatus.

The lot information of a part records the lot number assigned when the part is shipped. When a failure occurs due to a lot defect, there is a high possibility that parts manufactured in the same lot as the part causing the failure or in a lot close to the lot of the part causing the failure cause a failure. Therefore, a recall is highly necessary. Therefore, in FIG. 2, it is desirable to combine apparatus installation information (apparatus ID information and installation location information) recorded by a constituent part database 33 of the management server 30 to specify the apparatus in which the part requiring recall is assembled.

The replacement type information of a part records two part replacement reasons, that is, "periodic" indicating a periodic replacement and "failure" indicating a failure replacement. Since the failure replacement is more urgent, it is effective in a case where the priority of replacement is determined by the influence of an inventory status of the part or the like.

Since the part record in the constituent part database 24 remains even after the part is removed from the apparatus ("status" is "state 4"), the recording of the replacement type information is useful also at the time of information collection, which investigates a relationship between a service life of the part and an environment at a customer site.

The time information about a time when a part is assembled into an apparatus and the number-of-day information about how many days a part can be used can be used as information for determining whether a periodic replacement of a part is necessary. For example, when "insertion date and time" + "replacement interval" - "current date and time" ≤ "notified recommended number-of-day for part replacement", a periodic replacement of the part is necessary.

Returning to FIG. 1, the automatic analyzer communication unit 22 can communicate with a management server communication unit 35. For example, information in the constituent part database 24 of the automatic analyzer 20 is transmitted to the management server 30.

The management server 30 includes a management server control unit 31, a management server storage unit 32 including the constituent part database 33, an analysis unit 34, and the management server communication unit 35.

The management server control unit 31 includes a CPU, a RAM, and a ROM, and can load a program stored in the ROM into the RAM and execute the program. The management server control unit 31 is connected to other units, and can exchange data with the other units and relay data between the units.

The management server storage unit 32 includes a recording medium such as a flash memory, and the management server storage unit 32 includes the constituent part database 33 similarly to the automatic analyzer 20. Since information recorded in the constituent part databases 24 of the automatic analyzers 20 operating at all the customer sites is aggregated in the constituent part database 33 of the management server 30, it is possible to confirm information about all the constituent parts (the analysis devices 25) of the automatic analyzers 20 from the constituent part database 33 of the management server 30.

In addition to information recording part records of the constituent part database 24 of the automatic analyzer 20, the constituent part database 33 of the management server 30 may record information necessary for using the aggregated information, such as the apparatus installation information described above.

The analysis unit 34 can analyze information received from the management server control unit 31 and transmit a result to the management server control unit 31. For example, the analysis unit 34 receives information of the constituent part database 33, analyzes whether there is a part requiring a replacement arrangement, such as a part that satisfies an "insertion date" + the "replacement interval" - the "current date and time" ≤ the "notified recommended number-of-day for part replacement" and has the "status" of state 1, and transmits a result to the management server control unit 31.

The management server communication unit 35 can communicate with the automatic analyzer communication unit 22 of the automatic analyzer 20 and a part center communication unit 41 of the part center 40. For example, the management server communication unit 35 acquires the information of the constituent part database 24 of the automatic analyzer 20.

The part center 40 includes the part center communication unit 41 and an ID tag creation unit 42.

The part center communication unit 41 can communicate with the management server communication unit 35. For example, part center communication unit 41 receives a part arrangement request from the management server 30.

The ID tag creation unit 42 creates a label including an ID tag to be attached to a packaging box of the analysis device 25 or the analysis device 25 when transporting the analysis device 25 for replacement. Here, two types of information are recorded in the ID tag.

The first type of information is information for specifying a removal part from the constituent part database 24 of the automatic analyzer 20 at the time of part replacement. In the embodiment, the information corresponds to the "part ID".

The second type of information is information for generating a record of an assembling part at the time of part replacement. When generating a record of an assembling part, basically, there are many pieces of information that can be recorded without information acquisition from the outside, based on a reference to the record information of the removal part and an input of a default value.

For example, since the assembling part immediately after replacement normally operates, the state information can always be recorded as "state 1".

However, there is also information difficult to record without information acquisition from the outside, such as the "lot number". By recording such information in the ID tag, automatic information acquisition can be achieved at the time of reading, and time and effort for the user's manual input can be reduced.

In the embodiment, the ID tag records the "part ID" and the "lot number" for the above reason. Regarding these types of information, the information to be recorded can be changed according to a use case.

For example, the "part name" and the "replacement interval" can be basically acquired from the record of the removal part, and normally, the information is not recorded in the ID tag.

However, when such information is changed at the time of occurrence of part upgrade, there is a possibility that information acquisition from the record of the removal part is difficult. In preparation for such a case, the information is recorded in the ID tag, and thus it is possible to reduce time and effort for information correction by the user's manual input.

However, an ID tag size depends on performance of a reading device of the ID tag read unit 12 of the part management apparatus and the amount of information recorded in the ID tag, and the ID tag size increases as the amount of information increases. Therefore, when the amount of information to be recorded is large, the use of attaching an ID tag to a part is difficult.

However, as described above, since the ID tag is required when managing the record information in the constituent part database 24 at the time of part replacement, it is desirable to attach the ID tag to the analysis device 25, and store in an external storage unit (constituent part database 24 or 33), and the ID tag may be attached only to a packaging box.

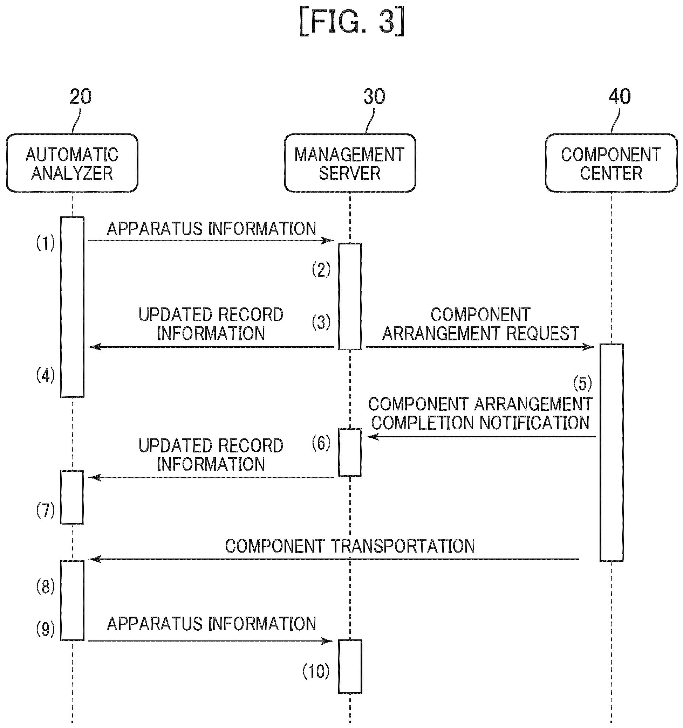

FIG. 3 illustrates an interface between part management systems in the embodiment until a part, which is assembled in the automatic analyzer 20 and operates normally, is removed by replacement.

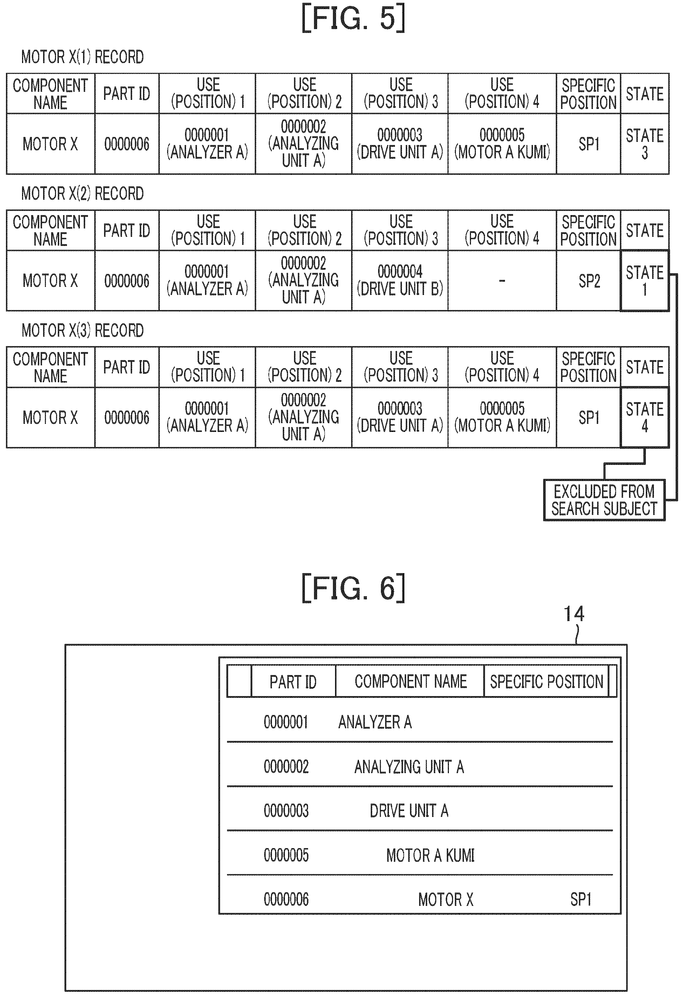

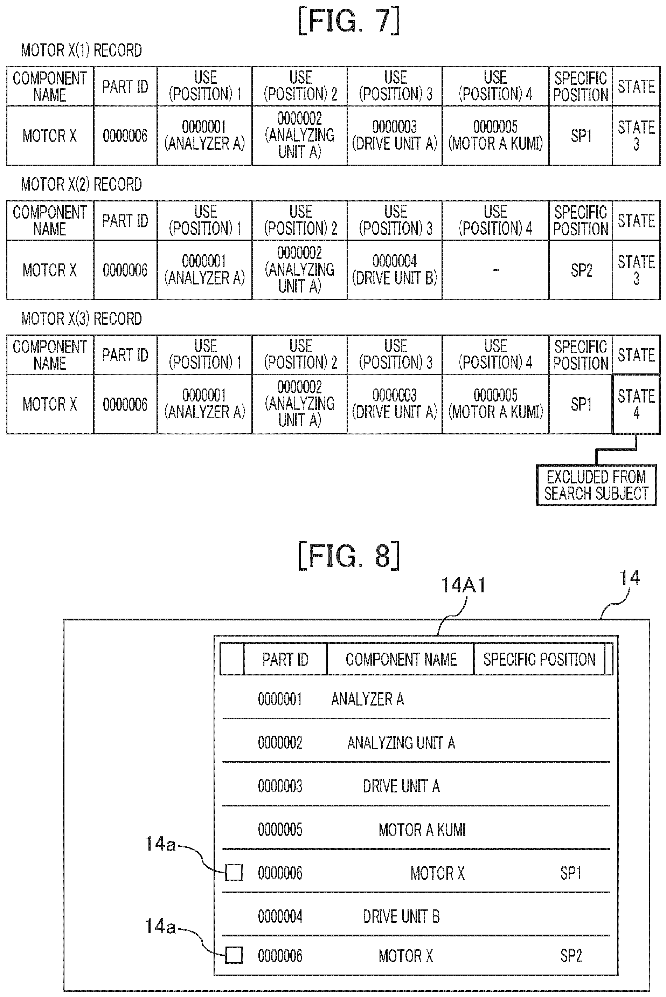

(1) First, the automatic analyzer control unit 21 of the automatic analyzer 20 periodically transmits apparatus information such as information in the constituent part database 24 of the automatic analyzer, alarm information, and part signal information to the management server 30 via the automatic analyzer communication unit 22. The step of (1), and steps of (4), (6), (7), (8), (9), and (10) described later correspond to a storage step of storing, for each of a plurality of analysis devices 25, an ID of the corresponding analysis device 25 and state information indicating a state of the analysis device 25, the ID and the state information being stored in association with each other.
(2) The management server 30 analyzes the apparatus information received from the automatic analyzer 20. When it is assumed that the number of days until a replacement time limit of a part reaches a set number-of-day or when it is assumed that the number of days is equal to or less than a predetermined threshold number of days and replacement at a close timing is necessary, or when a failure of the part is detected, the management server 30 updates the "status" of the corresponding part record in the constituent part database 33 of the management server 30 from "state 1" to "state 2", and records that an arrangement for the part is necessary.
(3) The information recorded by the constituent part database 33 of the management server 30 and information recorded by the constituent part database 24 of the automatic analyzer 20 need to be synchronized. At this timing, since the information in the constituent part database 33 of the management server 30 is the latest version, the information of the part record in which the "status" is updated in the constituent part database 33 of the management server 30 is transmitted to the automatic analyzer 20. The management server 30 transmits a part arrangement request to the part center 40 for the part whose "status" is updated to "state 2". The step (3) and the step (2) described above correspond to a reception step of receiving an replacement request for the analysis device 25.
(4) The automatic analyzer 20 reflects the record information received from the management server 30 in the constituent part database 24 of the automatic analyzer 20 (updates the "status").
(5) The part center 40 that receives the part arrangement request performs an arrangement for the part. When the part arrangement is completed, a part arrangement completion notification is transmitted to the management server 30, and the part is transported. In addition, a label including an ID tag created by the part center 40 is attached to at least one of a packaging box and the part at the time of part transport.
(6) The management server 30 updates the "status" of an arrangement completion part record in the constituent part database 33 of the management server 30 from "state 2" to "state 3" based on the part arrangement completion notification acquired from the part center 40. The information about the updated record is transmitted to the automatic analyzer 20.
(7) The automatic analyzer 20 reflects the record information received from the management server 30 in the constituent part database 24 of the automatic analyzer 20 (updates the "status").
(8) As soon as the arranged part reaches an installation location of the automatic analyzer 20, a user performs the part replacement. At this time, a screen as illustrated in FIG. 6 or FIG. 8, which will be described later, is displayed on the display unit 14 of the part management apparatus 10, and the user can perform the replacement work according to the display of the displayed screen. In addition, the constituent part database 24 of the automatic analyzer 20 updates the "status" of the record of the part removed from the apparatus by replacement from "state 3" to "state 4", and newly generates a record of the part newly assembled into the apparatus by the replacement. At this time, the "status" of the newly generated record of the assembling part is set to "state 1". The step of (8) corresponds to a display step of displaying, when the replacement request for the analysis device 25 is received in the reception step, the ID of the analysis device 25 whose state information satisfies a predetermined requirement and the first state information about a position where the analysis device 25 is used.
(9) The apparatus information including information in the constituent part database 24 of the automatic analyzer 20 in which the record of the part replacement is reflected is transmitted to the management server 30 at the timing of periodic information transmission similarly to (1).
(10) The management server 30 analyzes the received apparatus information similarly to the step of (2). At this time, the record information updated and newly generated in the constituent part database 24 of the automatic analyzer 20 by (8) is reflected in the constituent part database 33 of the management server 30.

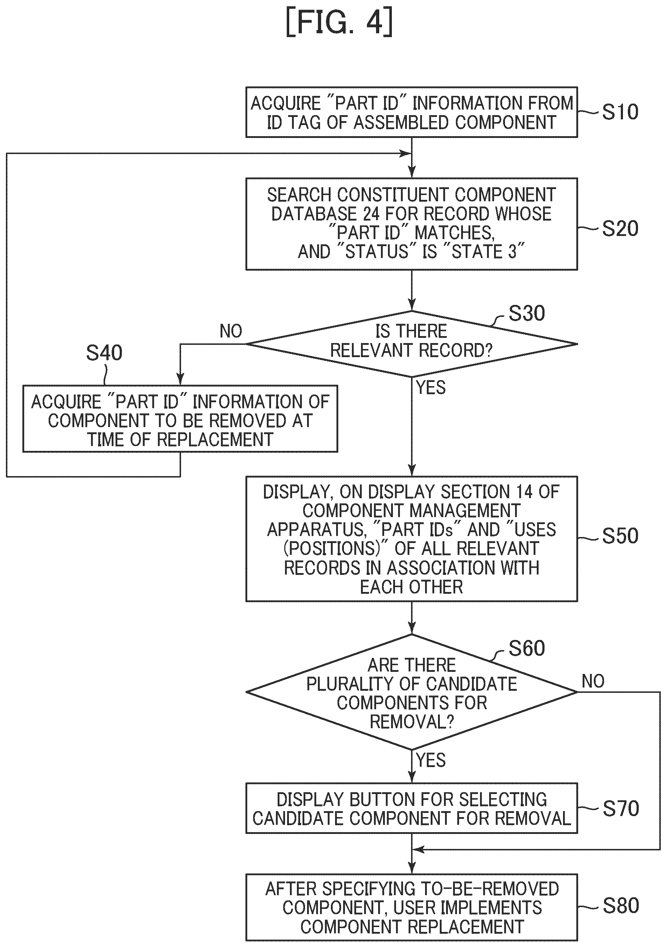

FIG. 4 illustrates a processing procedure of the part management apparatus 10 at the time of part replacement in the step of (8) described above.

First, the ID tag read unit 12 of the part management apparatus 10 acquires information recorded by an ID tag attached to a packaging box of a part to be assembled into an apparatus by replacement (S10).

In a normal part replacement, the same type of parts are replaced with each other, and the "part IDs" of a removal part and an assembling part coincide with each other. The "status" of a record of the removal part at the time of part replacement is updated to state 3 when the management server 30 receives an order arrangement completion notification from the part center 40.

Therefore, by retrieving a record, in which the "part ID" coincides with the "part ID" acquired from the ID tag and the "status" is "state 3", from the constituent part database 24 (S20), the removal part is specified.

However, for example, when the "part ID" is changed due to part upgrade, the "part ID" of the assembling part (ID tag) and the "part ID" of the removal part may not coincide with each other. Accordingly, the part management apparatus 10 determines whether there is a record that satisfies a requirement (S30). When it is determined that a record satisfying a retrieval requirement is present in the constituent part database 24 of the automatic analyzer 20, the process proceeds to step S50.

On the other hand, when it is determined that there is no record satisfying the retrieval requirement in the constituent part database 24, the removal part cannot be specified based on the "part ID" of the assembling part. In such a case, the removal part needs to be specified by another method. For example, the user manually inputs the "part ID" of the removal part. Alternatively, a change history of the "part ID" is recorded in the management server 30, and when the "part ID" of the assembling part is not in the constituent part database 24, the "part ID" coincident with that of the removal part before the change is acquired from the management server 30. The "part ID" of the part to be removed is acquired by these methods (S40), and the information is used to perform the retrieval again in the constituent part database 24 of the automatic analyzer 20 (S20), and the removal part is specified.

Here, in a case where there are a plurality of parts with the same "part ID" whose "status" is "state 3", there is a possibility that the one removal part cannot be specified just by the retrieval at the above requirements. Therefore, the part management apparatus 10 displays the "part ID" and the "use or position" information of all the records satisfying the requirements in association with each other on the display unit 14 (S50).

Next, the user determines whether there are a plurality of parts that are removal candidates displayed on the display unit 14 in the preceding step S50 (S60). When it is determined that there is only one record rather than multiple records, the part to be removed from the automatic analyzer 20 by replacement can be specified, and thus the process proceeds to step S80.

On the other hand, when a plurality of parts are displayed, a button for selecting, from the plurality of removal candidate parts, a part to be actually removed is displayed (S70).

After specifying one removal part, the user performs the part replacement (S80).

Next, an example of information in the constituent part database 24 and a display example on the display unit 14 will be described with reference to FIGS. 5 to 8.

First, an example will be described with reference to FIGS. 5 and 6 in which, when the "part ID" (0000008) of a motor X is acquired from an ID tag, records of three motors X are recorded in the constituent part database, and the ID of one analysis device 25 and the first state information about a position where the analysis device 25 is used are displayed on the display unit 14. FIG. 5 is a diagram illustrating an example of the record information, and FIG. 6 is a diagram illustrating an example of display of the display unit.

As illustrated in FIG. 5, a motor X (2) whose "status" is "state 1" and a motor X (3) whose "status" is "state 4" are not removal part candidates, and therefore are not displayed on the display unit 14.

On the other hand, since a motor X (1) is in "state 3" as illustrated in FIG. 5, a specification screen 14A is displayed on the display unit 14. On the specification screen, a part ID "0000001" in "use or position 1" and an "analyzer A" that is position information thereof, a part ID "0000002" in "use or position 2" and an "analysis unit A" that is position information thereof, a part ID "0000003" in "use or position 1" and a "drive unit A" that is position information thereof, a part ID "0000005" in "use or position 1" and a "motor A set" that is position information thereof, a part ID "0000006" of the "motor X" that is the corresponding analysis device 25 and the part name "motor X", and position information "SP1" are displayed so that the position can be specified step by step from the largest frame as illustrated in FIG. 6 . Accordingly, the user can easily specify an attachment position of the removal candidate part.

Next, a display example on the display unit 14 in a case where the records of the three motors X are recorded in the constituent part database when the "part ID" (0000008) of the motor X is acquired from an ID tag will be described with reference to FIGS. 7 and 8. FIG. 7 is a diagram illustrating an example of record information in a case where there are a plurality of removal part candidates. FIG. 8 is a diagram illustrating an example of display of the display unit in the case where there are a plurality of removal part candidates.

Similarly to FIG. 5, as illustrated in FIG. 7, the motor X (3) whose "status" is state 4 is not a removal part candidate, and therefore is not displayed on the display unit 14.

On the other hand, as illustrated in FIG. 7, the same information from "use or position 1" to "use or position 2" is recorded for both the motor X (1) and the motor X (2). However, for the motor X (1), the "use or position 3" is "0000003 (drive unit A)" and the "use or position 4" is "0000005 (motor A set)", and for the motor X (2), the "use or position 3" is "0000004 (drive unit B)". The motor X (1) and the motor X (2) are different in the "use or position". As described, even when parts having the same "part ID", use positions thereof in the apparatus do not coincide with each other.

Accordingly, on a specification screen 14A1 displayed on the display unit 14, the "use or position" is displayed in association with the "part ID", and thus the user can distinguish among removal candidate parts based on a difference in use position.

As a removal part specifying method, as illustrated in FIG. 8, a check box 14a is provided on the left side of the "part ID" of the removal candidate part, and the part to be removed can be specified by the user's selection.

Further, when an ID tag is directly attached to a part having a higher hierarchy, the ID tag is read and thus the removal candidate part can be limited to parts whose "part ID" same as the ID tag are included in the "use or position".

In the example in FIG. 7, since the use or position information 1 to 2 is common, if an ID tag is directly attached to any part of "0000003 (drive unit A) ", "0000005 (motor A set)", or "0000004 (drive unit B)", it is possible to specify which motor X is to be removed by reading the ID tag.

At the time of part replacement, the "part ID" and the "use or position" in the record of the removal part specified by a method similar to that in FIG. 6 or FIG. 8 are displayed on the display unit 14 in association with each other, so that a support screen presenting a replacement position to the user is set.

In the above-described embodiment, the state information of the removal part at the time of part replacement being "state 3" is used for specifying the removal part. However, in a case of replacing a kit product that is sold with a plurality of types of parts collectively or replacing a part that is sold with other parts of the same type, there is a possibility of replacing the part collectively with other parts, and the state information of the removal part is not necessarily "state 3".

It is possible to deal with such a case by freely setting the retrieval requirements such that parts in any one or more of "state 3", "state 1" and "state 2" are removal part candidates.

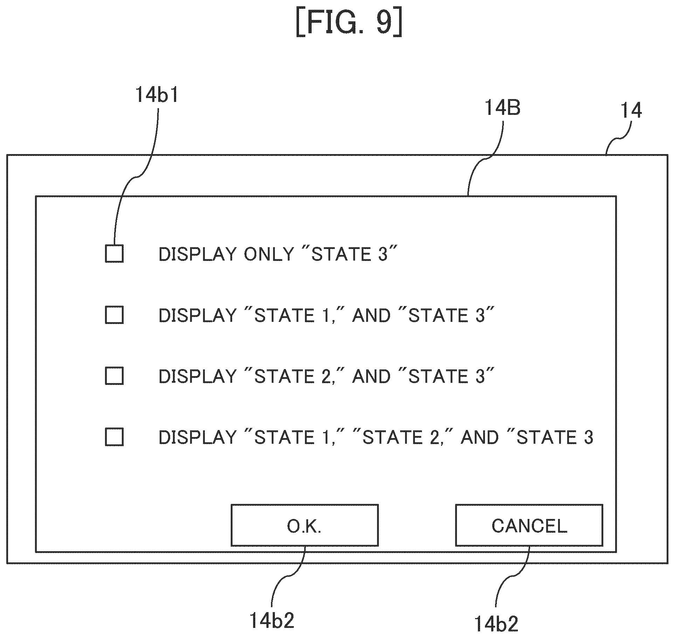

Therefore, as illustrated in FIG. 9, it is desirable that the part management apparatus control unit 11 causes the display unit 14 to display, on a selection screen 14B, one or more of the ID of the analysis device 25 in "state 1" in which the analysis device 25 is normally assembled and the second state information about a position where the analysis device 25 is used, and the ID of the analysis device 25 in "state 2" for waiting for the arrangement of the analysis device 25 and the third state information about a position where the analysis device 25 is used.

When any one of check boxes 14b1 is selected and an O.K. button 14b2 is pressed on the selection screen 14B, the ID and the position of a part in the "state" whose check box 14b1 is checked are displayed. When a cancel button 14b3 is selected, the selection screen 14B is closed.

However, a part in "state 4" is already removed and its replacement product is not assembled in the apparatus, and therefore will not be a removal part candidate in the future. Therefore, it is desirable that the part management apparatus control unit 11 hides and does not display, on the display unit 14, the ID of the analysis device 25 that is already not assembled in the apparatus and the fourth state information indicating the position where the analysis device 25 is used.

In the above-described embodiment, a case in which the part management system 1 includes the part management apparatus 10, the automatic analyzer 20, the management server 30, and the part center 40 is described. Alternatively, in the part management system 1, the automatic analyzer 20 may include one or more configurations of the part management apparatus 10 or the management server 30, and the automatic analyzer 20 may be a separate apparatus.

In this case, the automatic analyzer may include: two or more analysis devices 25 used for measurement of physical properties; a display unit 25a that displays information about the automatic analyzer; the automatic analyzer storage unit 23 that stores, for each of the plurality of analysis devices 25, an ID of the corresponding analysis device 25 and state information indicating a state of the analysis device 25, the ID and the state information being stored in association with each other; an input unit 25b that receives a replacement request for the analysis device 25; and the automatic analyzer control unit 21 that, when the input unit 25b receives a replacement request for the analysis device 25, causes the display unit 25a to display the ID of the analysis device 25 whose state information satisfies a predetermined requirement and first state information about a position where the analysis device 25 is used.

Next, effects of the embodiment will be described.

The part management system 1 for managing a plurality of analysis devices 25 used in an apparatus according to the embodiment described above includes: the display unit 14 that displays information about the part management system 1; the automatic analyzer storage unit 23 that stores, for each of the plurality of analysis devices 25, an ID of the corresponding analysis device 25 and state information indicating a state of the analysis device 25, the ID and the state information being stored in association with each other; the input unit 13 that receives a replacement request for the analysis device 25; and the part management apparatus control unit 11 that, when the input unit 13 receives a replacement request for the analysis device 25, causes the display unit 14 to display the ID of the analysis device 25 whose state information satisfies a predetermined requirement and first state information about a position where the analysis device 25 is used.

Accordingly, even in a case where parts of the same type are used in a plurality of uses or positions, since state information of the analysis device 25 is used and information for specifying the analysis device 25 to be removed at the time of replacement and position information are displayed to a user, it is possible to easily specify the analysis device 25 to be removed.

Such a part that can be managed by the part management system 1 is not limited to the analysis device 25 of the automatic analyzer 20 used in clinical test, and can be applied to various multifunction devices in which constituent parts change due to replacement of consumable parts, repair, and the like, such as devices on ships and aircrafts used in the field of transportation and machine tools used in manufacturing and processing parts, and efficient management of constituent parts can be achieved.

In addition, since it is assumed that the predetermined requirement is a state where preparation of a replacement product of the analysis device 25 is completed and the replacement is ready to be implemented, information about the part that the replacement work can be started immediately can be displayed, and thus it is possible to implement display that is more easily understood by the user.

Further, the part management apparatus control unit 11 causes the display unit 14 to display one or more of the ID of the analysis device 25 in a state in which the analysis device 25 is normally assembled and second state information about a position where the analysis device 25 is used, and the ID of the analysis device 25 in a state of waiting for an arrangement of the analysis device 25 and third state information about a position where the analysis device 25 is used. Accordingly, states at other positions can be grasped, and replacement positions can be easily grasped even in a case of replacing a kit product that is sold with a plurality of types of parts collectively or replacing a part that is sold with other parts of the same type.

Since the ID of the analysis device 25 that is already not assembled in the apparatus and fourth state information indicating a position where the analysis device 25 is used are hidden without being displayed on the display unit 14 by the part management apparatus control unit 11, unnecessary information is not displayed and only information necessary for the user is displayed. Accordingly, it is more easy to grasp how to perform the replacement work.

Further, in a case where a replacement time limit of the analysis device 25 normally operating in the apparatus is reached or approaches, or in a case where a failure occurs, the second state information is updated to the third state information. Accordingly, it is possible to automatically arrange a replacement part without requiring the user to update the information, and it is possible to restrict an occurrence of missed and delayed replacement, and the like.

In a case where the preparation of the replacement product of the analysis device 25 is completed and the replacement is ready to be implemented, the third state information is updated to the first state information. Accordingly, it is unnecessary for the user to update the information, and a further reduction in burden can be achieved.

Further, when the replacement of the analysis device 25 is completed, the first state information is updated to the fourth state information. Accordingly, it is unnecessary for the user to update the information, and since unnecessary information is prevented from being displayed, it is easier for the user to grasp only necessary information.

When the replacement of the analysis device 25 is completed, new state information is added. Accordingly, it is unnecessary for the user to update the information.

### <Others>

The invention is not limited to the embodiments described above, and various modifications and applications are possible. The embodiments described above are described in detail for easy understanding of the invention, and are not necessarily limited to those having all the configurations described above.

### Reference Signs List

1: part management system
10: part management apparatus
11: part management apparatus control unit (control unit)
12: ID tag read unit
13: input unit (reception unit)
14: display unit
14A, 14A1: specification screen
14a, 14b1: check box
14B: selection screen
14b2: O.K. button
14b3: cancel button
15: part management apparatus communication unit
20: automatic analyzer
21: automatic analyzer control unit
22: automatic analyzer communication unit
23: automatic analyzer storage unit
24: constituent part database of automatic analyzer
25: analysis device (part)
25a: display unit
25b: input unit
30: management server
31: management server control unit
32: management server storage unit
33: constituent part database of management server
34: analysis unit
35: management server communication unit
40: part center
41: part center communication unit
42: ID tag creation unit

## Claims

1. A part management system for managing a plurality of parts used in an apparatus, the part management system comprising:
a display unit that displays information about the part management system;
a storage unit that stores, for each of the plurality of parts, an ID of the corresponding part and state information indicating a state of the part, the ID and the state information being stored in association with each other;
a reception unit that receives a replacement request for the part; and
a control unit that, when the reception unit receives a replacement request for the part, causes the display unit to display an ID of the part whose state information satisfies a predetermined requirement, and first state information about a position where the part is used.

2. The part management system according to claim 1, wherein
the predetermined requirement is that preparation of a replacement product of the part is completed and replacement is ready to be implemented.

3. The part management system according to claim 2, wherein
the control unit causes the display unit to display one or more of an ID of the part that is assembled normally and second state information about a position where the part is used, and an ID of the part in a state of waiting for an arrangement of the part and third state information about a position where the part is used.

4. The part management system according to claim 3, wherein
the control unit hides without displaying, on the display unit, an ID of the part that is already not assembled in the apparatus and fourth state information indicating a position where the at part is used.

5. The part management system according to claim 3, wherein
in a case where a replacement time limit of the part operating normally in the apparatus is reached or approaches, or in a case where a failure occurs, the second state information is updated to the third state information.

6. The part management system according to claim 3, wherein
in a case where preparation of a replacement product of the part is completed and replacement is ready to be implemented, the third state information is updated to the first state information.

7. The part management system according to claim 4, wherein
in a case where replacement of the part is completed, the first state information is updated to the fourth state information.

8. The part management system according to claim 3, wherein
in a case where replacement of the part is completed, new state information is added.

9. The part management system according to claim 1, wherein the apparatus is an automatic analyzer that performs measurement of a physical property of a biological sample.

10. An automatic analyzer for causing a biological sample and a reagent to react with each other and measuring a physical property of a reacted reaction liquid, the automatic analyzer comprising:
two or more parts used for measurement of the physical property;
a display unit that displays information about the automatic analyzer;
a storage unit that stores, for each a plurality of parts, an ID of the corresponding part and state information indicating a state of the part, the ID and the state information being stored in association with each other;
a reception unit that receives a replacement request for the part; and
a control unit that, when the reception unit receives a replacement request for the part, causes the display unit to display an ID of the part whose state information satisfies a predetermined requirement, and first state information about a position where the part is used.

11. A part management method for managing a plurality of parts used in an apparatus, the part management method comprising:
a storage step of storing, for each of the plurality of parts, an ID of the corresponding part and state information indicating a state of the part, the ID and the state information being stored in association with each other;
a reception step of receiving a replacement request for the part; and
a display step of, when a replacement request for the part is received in the reception step, displaying an ID of the part whose state information satisfies a predetermined requirement, and first state information about a position where the part is used.
